# EUROPEAN PATENT SPECIFICATION

(11) **EP 2 635 267 B1**
(45) Date of publication and mention of the grant of the patent: **12.06.2019**
(21) Application number: 11837679.7
(22) Date of filing: 31.10.2011
(51) Int. Cl.: A61K 31/122, A61P 3/06, A61P 3/10, A61P 9/00, A61P 9/12, A61P 25/24

(54) **VITAMIN MK-7 FOR USE IN INCREASING HIGH DENSITY LIPOPROTEIN**
VITAMIN MK-7 ZUR VERWENDUNG BEI DER ERHÖHUNG VON LIPOPROTEIN HOHER DICHTE
VITAMINE MK-7 POUR SON UTILISATION DANS L'AUGMENTATION DE LA LIPOPROTÉINE DE HAUTE DENSITÉ

(30) Priority: 01.11.2010 IN 3024MU2010
(43) Date of publication of application: 11.09.2013
(73) Proprietor: Viridis Biopharma Pvt Ltd., Maharashtra (IN)
(72) Inventor: DILIP, Mehta, Mumbai 400006 (IN); RAMA, Vaidya, Mumbai (IN); DE SOUZA, Anselm, Mumbai 400080 (IN); YOGESH, Dound, Mumbai 400060 (IN); ASHOK, Vaidya, Mumbai (IN)
(74) Representative: Rees, Kerry
(86) International application number: PCT/IN2011/000751
(87) International publication number: WO 2012/059942

(56) References cited:
- EP-A1- 2 130 533
- WO-A2-2008/006607
- WO-A2-2010/103545
- US-A1- 2006 166 948
- US-A1- 2008 305 096
- US-A1- 2010 130 618
- REES K ET AL: "Is vitamin K consumption associated with cardio-metabolic disorders? A systematic review", MATURITAS, ELSEVIER, AMSTERDAM, NL, vol. 67, no. 2, 1 October 2010 (2010-10-01), pages 121-128, XP027288882, ISSN: 0378-5122 [retrieved on 2010-06-17]
- Makiko Yoshida ET AL: "Phylloquinone intake, insulin sensitivity, and glycemic status in men and women", J. Clin. Nutr., 1 January 2008 (2008-01-01), pages 210-215, XP055339486, Retrieved from the Internet: URL:http://ajcn.nutrition.org/content/88/1 /210.full.pdf [retrieved on 2017-01-26]
- GAST G C M ET AL: "A high menaquinone intake reduces the incidence of coronary heart disease", NMCD. NUTRITION METABOLISM AND CARDIOVASCULAR DISEASES, MILAN, IT, vol. 19, no. 7, 1 September 2009 (2009-09-01), pages 504-510, XP026352079, ISSN: 0939-4753 [retrieved on 2009-02-28]
- Ellen Cranenburg ET AL: "Vitamin K: The coagulation vitamin that became omnipotent", Thrombosis and Haemostasis, 1 July 2007 (2007-07-01), pages 120-125, XP055339457, Retrieved from the Internet: URL:http://www.anaboliclabs.com/User/Docum ent/Articles/Vitamin%20K/2.%20Cranenburg,% 20Vitamin%20K2,%202007.pdf [retrieved on 2017-01-26]
- ANONYMOUS: "Vitamin K2 may lower serum cholesterol", AMERICAN JOURNAL OF NURSING, vol. 98, no. 6, June 1998 (1998-06), page 54, XP008183008,
- MALLIANI ET AL.: 'Sympathovagal balance: a reappraisal.' CIRCULATION vol. 98, 1998, pages 2640 - 2643, XP055302997 Retrieved from the Internet: <URL:http://circ.ahajoumals.org/content/98/ 23/2640.2.full.pdf+html>
- MARK.: 'Clinical electrocardiography and arrhythmias.' HARVARD-MIT DIVISION OF HEALTH SCIENCES AND TECHNOLOGY 2004, XP055337217 Retrieved from the Internet: <URL:http://ocw.mit.eduicourseslhealth-scie nces-and-technology/hst-542j-quantitative-p hysiology-org an-transport-systems-spring-20041readings/c lin_elec_card.pdf>

## Description

### Field of the Invention:

Described herein is a method of increasing HDL in mammals by administering effective amounts of vitamin MK-7 or other vitamin K analogues and their derivatives. The invention relates to therapeutically effective amounts of vitamin MK-7 for use in a method of increasing High Density Lipoprotein (HDL) in a mammal in need thereof.

### Background of Invention:

Sudden cardiac death and Myocardial Infraction (MI) are major causes of death in both developed and developing countries. In the US prevalence of MI lies between 300,000 and 500,000 deaths every year [1]. The Autonomic Nervous System (ANS) plays an important role in the pathophysiology of conduction system of heart and thus a major disturbance in ANS can result in sudden cardiac death. The current available treatment modalities are based on the correction in the autonomic dysfunction. While doing so most of these drugs themselves produce serious side effects such as prolongation of QT interval. Such QT change is reason for the recall of several drugs [45]. There is need for safe intervention in these serious life threatening conditions. It was serendipitously observed that vitamin K2-7, (MK-7), shows promising result in the correction of sympathovagal balance.

Sympathetic Nervous System (SNS) and Parasympathetic Nervous System (PNS) are tonically active. This means that there is continuous nervous input from both the systems to a given tissue.

In other words, each system may enhance or inhibit tissue activity. This characteristic of the ANS improves its ability to more precisely regulate a tissue's function.

The balance between these two systems is critical in maintaining the homeostasis in proper state. Some time the PNS may not always work well like in case of Anxiety Disorders, Aging, Obesity etc [2-4] and to counter this deficiency SNS may be working overtime. This creates an unhealthy autonomic balance. Research shows that this imbalance in nervous system can be associated with emotional stress as well as cardiac disease.

It is known that the PNS can change faster than the SNS. Thus, as the SNS starts to mediate a stress response the PNS immediately begins to counter it. If the PNS were not faster than the SNS, then any stress response could send the heart into tachycardia and onto ventricular fibrillation before the PNS could act to prevent it.

The PNS through the Vagus have the main controlling influence on respiratory activity. PNS input to the heart is through fibers that synapse deep in the myocardium. SNS influence on the heart is through surface synapses. Due to this arrangement the PNS nerves are more critical to preventing heart damage (i.e., infarct, ischemia, or cardiomyopathies). Since the PNS nerves are faster to respond, it is usually the branch that is first to indicate changes in health status anywhere in the body.

The ANS regulate the conduction system within the heart. It is due to the stimulation of SNS & PNS, there is generation of electrical conduction which record as various waves on log paper.

### ECG

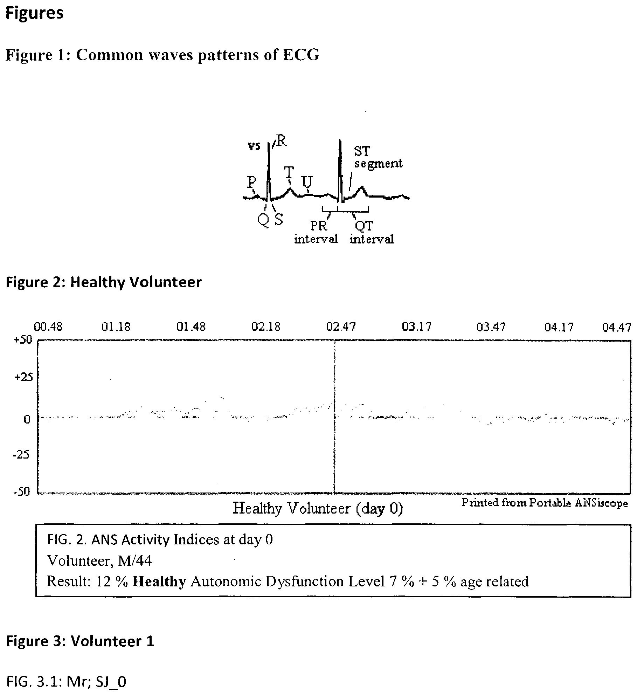

ECG is a graphical representation of the electrical activity of the heart. P, QRS complex and T are common waves produced during this electrical activity, as shown in Figure 1.

They represent:
- P wave: represents the depolarization impulse across the atria
- Q, R and S complex: all these three waves represent the ventricular depolarization (the downward stroke followed by an upward stroke is called Q wave and an further upward stroke is called R wave and any downward stroke preceded by an upward stroke is called S wave)
- T wave: represents the repolarization of the ventricles

Apart from these waves few intervals which are observed during this electrical activity are QT, QTc, PR and RR interval.
- QT interval: QT- interval provides a measure of ventricular repolarization and is determined by the balance of the repolarizing inward sodium and calcium currents, and the outward potassium and chloride currents. If there is cardiac vagal dysfunction it will result in prolongation of QT interval and if there is stimulation of cardiac vagal nerve it will result in the shortening of the QT interval. QT interval represents both the dispersion and the lengthening of the action potential duration and correlates directly with the left ventricular mass. Thus in case of cardiac hypertrophy there is increase in ventricular mass and prolongation of ventricular repolarization, so the QT interval is prolonged. During animal toxicity studies if the test product shows prolongation of QT interval then that product is considered as having cardiotoxic properties and may harm the cardiovascular system.
- QTc: In humans and large animals, QT interval varies strongly and inversely with heart rate. For these reasons it is recommended to correct the QT interval which is independent of heart rate. This is called as corrected QT interval (QTc). Conditions such as Coronary artery diseases (CAD), Cardiomyopathy, severe Bradycardia, High-Grade AV Block, Anti-Arrhythmics, Psychotropic Drugs, Hypocalemia and electrolyte imbalance, Congenital Long QT Syndrome, Hypothyroidism, leads to prolongation of QTc. From toxicological point of view it is important to see whether there is any prolongation of QTc.
- RR interval: RR interval is the interval from the peak of one QRS complex to the peak of next QRS complex. It is used to assess the ventricular rate / Heart Rate. Heart rate may increase by increase in sympathetic activity and decrease in parasympathetic activity and inversely true for decrease in Heart Rate. This sympathovagal balance is essential in maintaining Heart Rate and is believed to be reflected in the beat-to-beat changes of the cardiac cycle.
- PR interval: PR interval represents the time the impulse takes to reach the ventricles from the sinus node. Sympathetic activity increase shall lead to decrease in PR interval and parasympathetic activity increase to prolongation of PR interval.

### Sudden Cardiac Death and Myocardial Infraction

Sudden cardiac death could be outcome of increased sympathetic activity and decreased parasympathetic activity. Increase in sympathetic activity leads to the increase in heart rate and tachycardia [6-8]. Reduction in sympathetic over-activation by beta blocker and calcium channel blocker protect against arrhythmias [9-10]. On the other hand parasympathetic activity is preventive in case of arrhythmias and sudden cardiac death. Early study done by Eckberg *et al* [11] found that there is remarkable change in the parasympathetic activity in most of advanced diseased states. Some population based studies have linked the QT interval on a 12-lead electrocardiogram (ECG) with an increased risk of ventricular arrhythmias and sudden cardiac death in patients with coronary artery disease [12-14] and even in the general population [15-17] Sudden cardiac death is often the first and only manifestation of coronary artery disease and cardiac arrhythmias. Most common treatment of choice is class III antiarrhytmic drugs. Many of class III antiarrhythmic drugs show adverse effect such as prolongation of QT interval, which leads to life threatening tachyarrhythmia, torsades de pointes (i.e., polymorphic ventricular tachycardia in which the QRS waves seem to "twist" around the baseline), and leads to the increase cardiac mortality [18,19]. This imbalance in sympathovegal tone can be measured by Heart Rate Variability (HRV). HRV primarily reflects tonic vegal activity.

### Heart rate variability (HRV)

HRV is a measure of variation in the heart rate. Decrease in HRV is now considered as a strong predictor of Myocardial Infraction (MI), Arrhythmias and sudden Cardiac Death, which could be a result of insufficient adaptation of the ANS [20,21]. Whereas higher HRV is indicative of good adaptation by ANS. The most widely used methods can be grouped under time-domain ('how much variability') and frequency-domain ('how is variability caused?').

Heart Rate Variability (HRV) is considered as a measure of sympathovegal tone [21, 20]. It represents the most predominant quantitative markers of the autonomic tone. Sympathovegal tone will change with changing physiological and pathological conditions. HRV can be increased with respect to increase in physiological state such as endurance exercise and might be modulated by different body positions, sleep, etc. Pathological conditions like type 2 diabetes mellitus [22], high blood pressure [23], gastroesophageal reflux disease (GERD) [24], IBS [25], depression [26], COPD [27], etc, can alter the Sympathovegal tone and decrease the HRV.

Various studies of HRV, Billman *et al* [28 29], Kleiger *et al* [30], Sztajzel *et al* [31], have concluded that a higher HRV is reflection of compensated heart with good function. Any Autonomic imbalance/ disturbance will show up as lower HRV. Studies of Collins *et al* [32], Gianaros *et al* [33], strengthen the observations of changes in HRV related to vulnerability to sudden cardiac death.

HRV is commonly used to access the sympathovegal balance but it has some limitations. It has been found that HRV is of limited use in the certain conditions such as ectopic beats and artifacts, heart transplants, presence of arrhythmias and pacemakers which leads to false interpretation of HRV. HRV combined with ventricular ectopic beats, signal-averaged ECG, or left ventricular function results in 30 % - 50% increase in prediction power in sudden cardiac death and Myocardial Infraction.

### Cardiac output:

Cardiac output is the volume of blood being pumped by the ventricle in a minute. An average cardiac output would be 5 L/min for a human male and 4.5 L/min for a female. Cardiac output is a symbol of function of heart. Every cell in the body needs oxygen and nutrients for there functioning. If the cells are working hard, with a high metabolic oxygen demand then the Cardiac Output is raised to increase the supply of oxygen to the cells. Apart from pumping of heart cardiac output is also regulated by the vascular resistance.

Cardiac Output increases when there is an increase in heart rate (HR), Change of posture, increased SNS activity, and decreased PNS activity. Contrary to this Vitamin K2-7 has decreased sympathetic activity and increased cardiac output.

### Natural Products:

Thus ANS activity needs to be kept in balanced state, to maintain proper internal homeostasis. It has been seen that ANS activity can be influenced by many natural products such as CoQ10, Green tea, Capsicin, Bezoar, Glycrrhiza, n-3 PUFAs, Curcuminoids, Epigallocatechin gallate, Kava extract etc [34-44] Products with combination's of this ingredients have been used for the control of ANS activity; e.g. CCGC (combined capsicin, green tea, and chichen essence tablets) [35]. ANS activity of these ingredients has been questioned in many trials and several publications [39-44].

There are several patent addressing treatment of autonomic dysfunction through various modalities including receptor inhibition [US 7626015], gangliosides [US5190925], electrical modulation [US7363076], Malto-oligosaccharide [US5965557], Decahydroquinoline-based anticholinergic agents [US5929087] and others [US5965557]. However, these patents & earlier described ingredients, mostly addressed ANS directly through the diseases associated with ANS. Measurement of ANS relates to a disease conditions. Tests to measure ANS dysfunction include Tilt table test where blood pressure is measured after the person, who is lying flat on a pivoting table, is tilted into an upright position. Valsalva maneuver can also be performed to measure the blood pressure. Also Sweat testing can be done either by acetylcholine stimulation or by dye method. Other tests may be done to check for disorders that can cause the autonomic disorder. Present investigation used direct ANS activity through US FD approved instrumentation. Inventors along with the investigator of the clinical and animal study observed that vitamin K2-7 (MK-7) produced marked effect on balancing the sympathovagal tone. This can lead to the use of MK-7 in amelioration and aid to protect treating of cardiovascular diseases, type 2 diabetes mellitus, high blood pressure, gastroesophageal reflux disease (GERD), IBS, depression , COPD, etc, where there is misbalancing of sympathovegal tone. US-A-2008/305096 discloses that vitamin K increases HDL-cholesterol. American Journal of Nursing, 98(6), 1998, p. 54 discloses that vitamin K2 lowers total cholesterol and increases the ratio HDL-C/LDL-C. There is no significant change in HDL-C.

### Summary of the Invention

In one aspect there is provided, therapeutically effective amounts of vitamin MK-7, for use in a method of increasing HDL in a mammal in need thereof.

### Brief Description of Drawings:

Figure 1: ECG with commonly observed wave patterns.
Figure 2: ANS activity indices at day 0 in a healthy volunteer.
Figure 3.1: ANS activity indices at day 0 in volunteer 1.
Figure 3.2: ANS activity indices at day 62 in volunteer 1.
Figure 4.1: ANS activity indices at day 0 in volunteer 2.
Figure 4.2: ANS activity indices at day 58 in volunteer 2.
Figure 5: ECG changes in rat model with 500µg of MK-7 (Figure 5.1: in Male Rats; Figure 5.2: in Female Rats)
Figure 6: ECG changes in rat model with 1000µg of MK-7 (Figure 6.1: in Male Rats; Figure 6.2: in Female Rats)

### Description of Invention:

### Effect on ANS (reference):

During the course of investigation with MK-7 (PCTllN2010100014), inventors of current application serendipitously discovered that MK-7 has effect on sympathetic and parasympathetic activity.

The investigator studied the effect of MK-7 on sympathovagal balance on 4 healthy male volunteers of age between 34 to 50 yrs for 2 months. ANS activity was measured and printed out in a graph form by the ANSiscope™, manufactured by DyAnsys a US based company. The instrument is US FDA approved patented instrument. This instrument assessed the autonomic function by calculating 500 Heart beats / RR intervals. After assessing this, the instrument will analyze the level of the dysfunction and the degree of autonomic neuropathy. For this reason they have classified autonomic dysfunction in 5 stages, namely Healthy (H), Early (E), Late (L), Advanced (A) and Most Advanced (MA). The percentage calculation values demarcating groups are: -11.5% to 11.5%: healthy group, 13.5% to 20%: early group, 23% to 50.99%: late group, 51% to 100%: advanced group and above falls in Most advance group [42].

Fig.2 is an ANS Activity Indices of a Male Healthy Volunteer at the age of 44 yrs. As can be seen, the measured Parasympathetic and Sympathetic activities are within -11.5 to 11.5 % which as classified above is for healthy group. Our human study examples, given later on, show the variabilities that exist in individuals at different stage of health in life.

The study parameters assessed were sympathetic and parasympathetic tone, change in blood pressure (orthostatic intolerance, measured at right. brachial artery), pulse (assessed by measuring the pulse at right. radial artery) and physical and general examination. Study was performed on subjects in relaxed supine position without any external stimulation, usually 2 hrs. after breakfast during morning time for 2 consecutive months.

During the study of the pharmacological activity and tolerability of vitamin MK-7, on ANS in Healthy volunteers, Inventors discovered that dysfunction of ANS if present was getting harmonized.

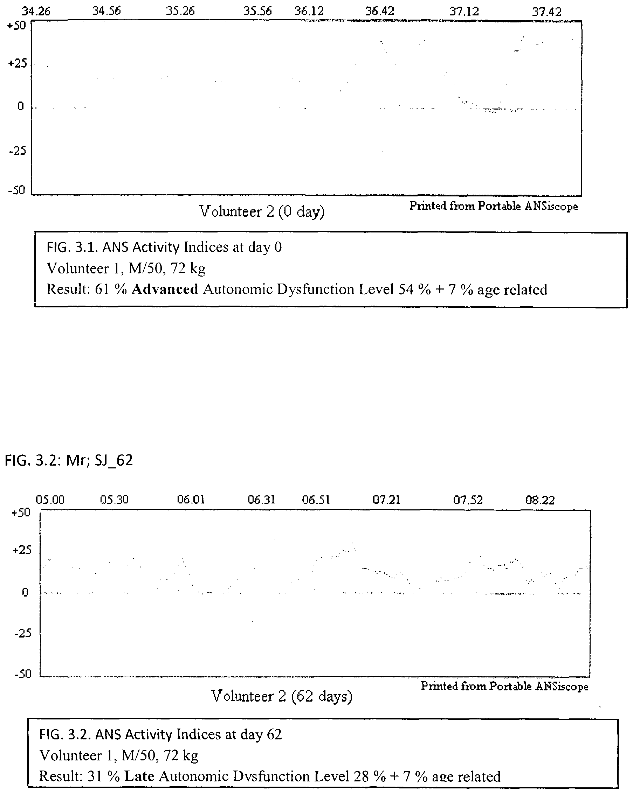

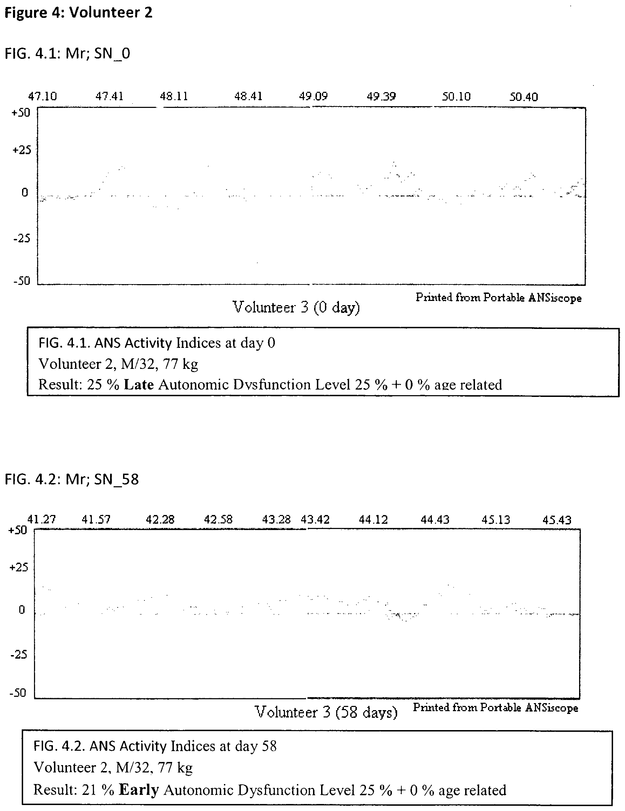

Subjects who have Blood pressure beyond the range of 140/90 (supine) and 100/70 (standing) mm Hg, and Sugar Fasting more than 250 mg% and with any major illness are ruled out of study. All subjects were checked for baseline parameters on 0 day. Vitamin K2-7 (MK-7) in dose of 350 µg was administered on 3^{rd} day after the baseline reading. MK-7 was continued for 2 months. Readings were taken on 3^{rd} day, 15^{th} day, 28^{st} day, 50^{th} day, 56^{th} day and at 62^{nd} day. It was observed that after 2 months of therapy with MK-7, there was a marked normalization in the sympathetic activity which was initially elevated, with little effect on parasympathetic activity (Figure 3 and 4). Other parameters remained unaffected.

This study provides window for the use of MK-7 in maintaining sympathovegal balance and can also be used as protective and preventive in cardiovascular diseases. Further studies with statistical sample size are needed to define efficacy of MK-7 in balancing ANS activity.

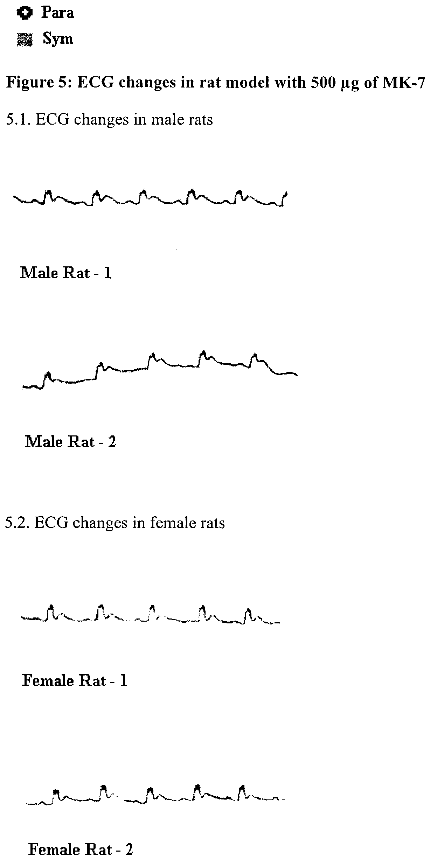

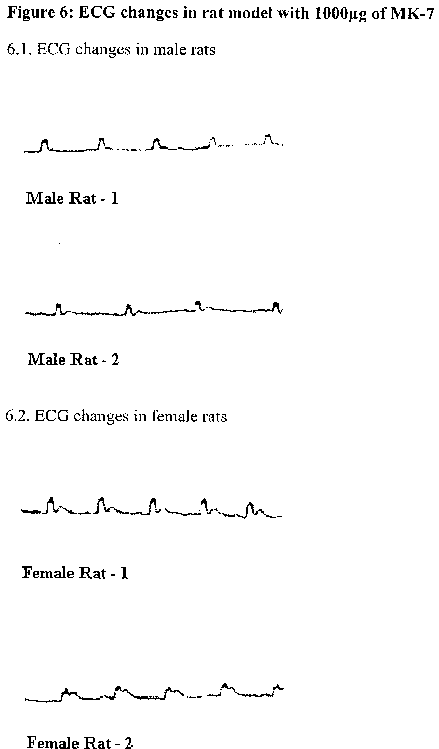

In another study on animal model during chronic toxicity study, it was discovered that MK-7 produces significant changes in ECG of rats. This discovery during basic pharmacology studies on MK-7 was investigated for its effect on Cardio Vascular System. It is during this study the investigator discovered the following findings (Figures 5 and 6).

The main parameter which was observed during chronic toxicity studies in rat ECG was the prolongation of QT interval. During toxicity studies if the test product shows prolongation of QT interval then that product is consider as having cardiotoxic properties and may harm the cardiovascular system. In this particular study the QT interval remained unaffected at all the three dose level i.e. at 100, 500, 1000 µg/kg.

In humans and large animals, QT interval varies strongly and inversely with heart rate. So it is advisable to have QTc. In this particular toxicity study conducted on rats, it did not cause any prolongation of QTc, whereas on another side the test drug causes moderate non-significant shortening of QTc.

QRS interval is indicative of ventricular depolarization and intra ventricular conduction time. In this particular toxicity study conducted on rats there was no increase in heart rate, which rules out supraventricular causes. There was significant shortening of QRS interval observed at 500, 1000 µg/kg dose level.

Also there were significant prolongation of RR interval complex was observed at 500, 1000 µg/kg dose level. These indicate that MK-7 causes prolongation of RR interval either by inhibition of sympathetic tone or stimulation of fast acting parasympathetic tone.

The PR interval was not affected to significant extent hence there is no influence of the drug on impulse transmission from atria to ventricle.

Thus the effects of MK-7 seem to be limited only to the ventricle since it is not reflected in changes in the PR interval.

### Effect on Cardiac Output parameters (reference):

During the yet another study conducted to assess the cardioprotective effect of vitamin K2-7, inventors of the current invention observe that the vitamin K2-7 improves the cardiac output parameters. Isoproterenol (ISO) induced cardiac injury model was selected for the study. It was observed that ISO induced cardiac output decrease in all the dose levels i.e. at 90 µg/kg, 450 µg/kg and 900 µg/kg. This ISO induced cardiac output decrease was found to be reversed in 90 µg/kg and 450 µg/kg dose test drug administered ISO injected rats. This effects was non-linear and was enhanced in 900 µg/kg dose.

### Left cardiac work (reference):

Effect of vitamin K2-7 on the left cardiac work has been evaluated in the same study. It was observed that administration of ISO leads to marked decrease in left cardiac work when measured 48h after ISO injection in comparison to pre ISO level i.e. control. This ISO induced left cardiac work decrease was found to be reversed to a great extent at all the dose levels i.e. at 90 µg/kg, 450 µg/kg and 900 µg/kg with ISO injected rats in comparison to ISO control. It was found that at the dose level of 900 µg/kg decrease in reversal was higher than other treatment groups.

### HDL (High Density Lipoprotein) cholesterol level (according to the invention):

In the same study it was also observed that vitamin K2-7 enhances the High Density Lipoprotein (HDL) level in all the dose levels studied i.e. at 90 µg/kg, 450 µg/kg and 900 µg/kg. Isoproterenol (ISO) induced cardiac injury model was selected for the study. In Isoproterenol control group significant decrease in HDL cholesterol level was observed in comparison to normal control group. HDL cholesterol level for the treated group increased in comparison to the ISO controlled group. The observed increase was greater with respect to the dose level increased.

### Blood Sugar level (reference):

In our previous patent PCTllN2010100014, we have described that vitamin K2, helps in reducing insulin resistances and bringing down blood sugar. Inventors, while conducting animal study have found that vitamin K2-7 helps in reducing blood sugar within 12 days of time at the dose level of 90 µg/kg, 450 µg/kg and 900 µg/kg. These findings are in consistent with our earlier findings reported in the PCTllN2010100014.

From the above novel, serendipitously observed finding it can be even proposed that the our cardioprotection claim is based upon several beneficial effects observed of Vitamin MK including deferent body system including ANS, Cardiac Output Parameters, Left cardiac work, HDL and Blood sugar level implying improved insulin sensitivity and enhance energy utilization.

### Definitions:

1. As used here in the term "ANS" refers to the Autonomic Nervous system.
2. As used here in the term "SNS" refers to the Sympathetic Nervous system.
3. As used herein the term "PNS" refers to the Parasympathetic Nervous system.
4. As used herein the term "SA Node" refers to the Sino Atrial Node.
5. As used herein the term "AV Node" refers to the Atrio Ventricular Node.
6. As used herein the term "HRV" refers to the Heart Rate Variability.
7. As used herein the term "BRS" refers to the baroreflex sensitivity.
8. As used herein the term "PUFAs" refers to the Poly unsaturated fatty acid.
9. As used herein the term "GERD" refers to the gastroesophageal reflux disease
10. As used herein the term "IBS" refers to the Inflammatory bowel syndrome.
11. As used herein the term "COPD" refers to the Chronic obstructive pulmonary diseases.
12. As used herein the term "CVD" refers to the Cardiovascular diseases.
13. As used herein the term ''AP" refers to the Action Potential.
14. As used herein the term "MK-7" refers to the Vitamin K2-7.
15. As used herein, Vitamin K analogues of the invention include but not restricted to vitamin K1, MK-4, MK-6, MK-8, MK-9 and other molecules having vitamin K activity.
16. As used here in the term "dromotropic effect" refers to increase in AP conduction velocity
17. As used herein, 'improving sympathovagal imbalance' encompasses improvement in the sympathetic & parasympathetic activity.
18. As used herein, 'promoting cardio protective effect' refers to the protective action of MK-7 on the cardiovascular system.
19. As used herein, 'increasing cardiac output' refers to the action of MK-7 on the cardiovascular system by enhancing cardiac output.
20. As used herein, 'increasing left cardiac work' refers to the action of MK-7 on the cardiovascular system by enhancing the left cardiac work.
21. Mammals as used in the disclosure refers to human beings.

### Case Studies:

### Example 1 (reference):

Male, 50 yrs, otherwise healthy was accessed for the ANS activity by the ANSiscope™, manufactured by DyAnsys a US based company on 28^{th} of April 2009. His percentage of dysfunction was 61% which fall under the 'Advance stage'. General and systemic examination including Blood Pressure (Supine) and Pulse was measured, which was in normal range. This was considered as a Baseline readings. On 3^{rd} day from his baseline reading he was then put on 1 cap per day of 350 µg, vitamin K2-7 for 2 months after breakfast at around 8 am in morning. He was accesses regularly initially at 1 weeks of interval in first month and at interval of 15 days in next month for ANS activity, B.P. and pulse. At the end of 2 months his percentage of ANS dysfunction changed from 61 % to 31%, which falls under 'Early stage'. Other parameters such as general and systemic examination including Blood Pressure (Supine), pulse were in normal range without any change from base line records. He was also feeling very energetic and can even work for longer time without any tiredness. (FIG. 3.1 and 3.2)

### Example 2 (reference):

Male, 32 yrs, otherwise healthy was accessed for the ANS activity by the ANSiscope™, manufactured by DyAnsys a US based company on 28^{th} of April 2009. His percentage of dysfunction was 25% which fall under the 'Late stage'. General and systemic examination including Blood Pressure (Supine) and Pulse was measured, which was in normal range. This was considered as a Baseline readings. On 3^{rd} day from his baseline reading he was then put on 1 cap per day of 350 µg, vitamin K2-7 for 2 months after breakfast at around 8 to 8.30 am in morning. He was accesses regularly for two months at interval of 15 days for ANS activity, B.P. and pulse. At the end of 2 months his percentage of ANS dysfunction changed from to 21%, which falls under 'Early stage' of dysfunction. Other parameters such as general and systemic examination including Blood Pressure (Supine), pulse were in normal range without any change from base line records. (FIG. 4.1 and 4.2).

### References:

1. Billman GE. Cardiac autonomic neural remodeling and susceptibility to sudden cardiac death: effect of endurance exercise training. Am J Physiol Heart Circ Physiol. 2009 Oct; 297(4):H1171-93. Epub 2009 Aug 14. PMID: 19684184.
2. Gazelle H, Druhen MJ. Anxious solitude and peer exclusion predict social helplessness, upset affect, and vagal regulation in response to behavioral rejection by a friend. Dev Psychol. 2009 Jul; 45(4):1077-96. PMID: 19586181
3. Wichi RB, De Angelis K, Jones L, Irigoyen MC. A brief review of chronic exercise intervention to prevent autonomic nervous system changes during the aging process. Clinics (Sao Paulo). 2009;64(3):253-8. PMID: 19330253.
4. Kral JG, Paez W, Wolfe BM. Vagal nerve function in obesity: therapeutic implications. World J Surg. 2009 Oct;33(10):1995-2006.
5. Inagaki M, Kawada T, Lie M, Zheng C, Sunagawa K, Sugimachi M. Intravascular parasympathetic cardiac nerve stimulation prevents ventricular arrhythmias during acute myocardial ischemia. Conf Proc IEEE Eng Med Biol Soc. 2005;7(1):7076-7079. PMID: 17281905.
6. Hu XR, Jiang H, Wen HZ, Lu ZB, Zhao DD, Huang CX. Effects of sympathetic nerve stimulation on connexin43 and ventricular arrhythmias during acute myocardial ischemia: experiment with rats. Zhonghua Yi Xue Za Zhi. 2008 Jun 24;88(24):1707-10. PMID: 19024544.
7. Dünser MW, Hasibeder WR. Sympathetic overstimulation during critical illness: adverse effects of adrenergic stress. J Intensive Care Med. 2009 Sep-Oct;24(5):293-316. Epub 2009 Aug 23. PMID: 19703817.
8. Zipes DP. Heart-brain interactions in cardiac arrhythmias: role of the autonomic nervous system. Cleve Clin J Med. 2008 Mar;75 Suppl 2:S94-6. PMID: 18540155.
9. Piccini JP, Hranitzky PM, Kilaru R, Rouleau JL, White HD, Aylward PE, Van de Werf F, Solomon SD, Califf RM, Velazquez EJ. Relation of mortality to failure to prescribe beta blockers acutely in patients with sustained ventricular tachycardia and ventricular fibrillation following acute myocardial infarction (from the VALsartan In Acute myocardial iNfarcTion trial [VALIANT] Registry). Am J Cardiol. 2008 Dec 1;102(11):1427-32. Epub 2008 Sep 11. PMID: 19026290.
10. Held PH, Yusuf S. Effects of beta-blockers and calcium channel blockers in acute myocardial infarction. Eur Heart J. 1993 Oct; 14 Suppl F: 18-25. PMID: 7903243.
11. Eckberg DL, Drabinsky M, and Braunwald E. Defective cardiac parasympathetic control in patients with heart disease. N Engl J Med 285: 877-883, 1971.
12. Hutton DM. The importance of routine QT interval measurement in rhythm interpretation. Dynamics. 2008 Fall;19(3):29-33. PMID: 18773713.
13. Shah SH, Pitt GS. Genetics of cardiac repolarization. Nat Genet. 2009 Apr;41(4):388-9. PMID: 19338079
14. Dekker JM, Schouten EG, Klootwijk P, Pool J, Kromhout D. Association between QT interval and coronary heart disease in middle-aged and elderly men. The Zutphen Study. Circulation 90: 779-785, 1994.
15. De Bruyne MC, Hoes AW, Kors JA, Hofman A, van Bemmel JH, Grobbee DE. QTc dispersion predicts cardiac mortality in the elderly: the Rotterdam Study. Circulation 97: 467-472, 1998.
16. Okin PM, Devereux RB, Howard BV, Fabsitz RR, Lee ET, Welty TK. Assessment of QT interval and QT dispersion for prediction of all-cause and cardiovascular mortality in American Indians: The Strong Heart Study. Circulation 101: 61-66, 2000.
17. Schouten EG, Dekker JM, Meppelink P, Kok FJ, Vandenbroucke JP, Pool J. QT interval prolongation predicts cardiovascular mortality in an apparently healthy population. Circulation 84: 1516-1523, 1991.
18. Riera AR, Uchida AH, Ferreira C, Ferreira Filho C, Schapachnik E, Dubner S, Zhang L, Moffa PJ. Relationship among amiodarone, new class III antiarrhythmics, miscellaneous agents and acquired long QT syndrome. Cardiol J. 2008;15(3):209-19. PMID: 18651412.
19. Sager PT. New advances in class III antiarrhythmic drug therapy. Curr Opin Cardiol 14: 15-23, 1999.
20. Vanderlei LC, Pastre CM, Hoshi RA, Carvalho TD, Godoy MF. Basic notions of heart rate variability and its clinical applicability. Rev Bras Cir Cardiovasc. 2009 Jun;24(2):205-17. PMID: 19768301.
21. Yildirim A, Soylu O, Da deviren B, Eksik A, Tezel T. Sympathetic overactivity in patients with left ventricular aneurysm in early period after anterior myocardial infarction: does sympathetic activity predict aneurysm formation? Angiology. 2007 Jun-Jul; 5 8(3):275-82. PMID: 17626980.
22. Carnethon MR, Golden SH, Folsom AR, Haskell W, Liao D. Prospective investigation of autonomic nervous system function and the development of type 2 diabetes: the Atherosclerosis Risk In Communities study, 1987-1998. Circulation. 2003 May 6;107(17):2190-5. Epub 2003 Apr 14. PMID: 12695289
23. Jones PP, Shapiro LF, Keisling GA, Jordan J, Shannon JR, Quaife RA, Seals DR. Altered autonomic support of arterial blood pressure with age in healthy men. Circulation. 2001 Nov 13; 104(20):2424-9. PMID: 11705819
24. Dobrek L, Nowakowski M, Mazur M, Herman RM, Thor PJ. Disturbances of the parasympathetic branch of the autonomic nervous system in patients with gastroesophageal reflux disease (GERD) estimated by short-term heart rate variability recordings. J Physiol Pharmacol. 2004 Jul;55 Suppl 2:77-90. PMID: 15608363.
25. Mazur M, Furgala A, Jablo ski K, Madroszkiewicz D, Cie ko-Michalska I, Bugajski A, Thor PJ. Dysfunction of the autonomic nervous system activity is responsible for gastric myoelectric disturbances in the irritable bowel syndrome patients. J Physiol Pharmacol. 2007 Aug;58 Suppl 3:131-9. PMID: 17901589.
26. Carney RM, Freedland KE, Veith RC. Depression, the autonomic nervous system, and coronary heart disease. Psychosom Med. 2005 May-Jun;67 Suppl 1:S29-33. PMID: 15953797.
27. Volterrani M, Scalvini S, Mazzuero G, Lanfranchi P, Colombo R, Clark AL, Levi G. Decreased heart rate variability in patients with chronic obstructive pulmonary disease. Chest. 1994 Nov; 106(5):1432-7. PMID: 7956396.
28. Billman GE, Hoskins RS. Time-series analysis of heart rate variability during submaximal exercise. Evidence for reduced cardiac vagal tone in animals susceptible to ventricular fibrillation. Circulation. 1989 Jul; 80(1): 146-57. PMID: 2567640.
29. Billman GE, Schwartz PJ, Gagnol JP, Stone HL.Cardiac response to submaximal exercise in dogs susceptible to sudden cardiac death. J Appl Physiol. 1985 Sep;59(3):890-7. PMID: 2865243.
30. Kleiger RE, Miller JP, Bigger JT Jr, Moss AJ.Decreased heart rate variability and its association with increased mortality after acute myocardial infarction. Am J Cardiol. 1987 Feb 1; 59(4):256-62. PMID: 3812275.
31. Sztajzel J. Heart rate variability: a noninvasive electrocardiographic method to measure the autonomic nervous system. Swiss Med Wkly. 2004 Sep 4; 134(35-36):514-22. PMID: 15517504.
32. Collins MN and Billman GE. Autonomic response to coronary occlusion in animals susceptible to ventricular fibrillation. Am J Physiol Heart Circ Physiol 257: H1886-H1894, 1989. PMID: 2603974
33. Gianaros PJ, Salomon K, Zhou F, Owens JF, Edmundowicz D, Kuller LH, Matthews KA. A greater reduction in high-frequency heart rate variability to a psychological stressor is associated with subclinical coronary and aortic calcification in postmenopausal women. Psychosom Med. 2005 Jul-Aug; 67(4):553-60. PMID: 16046367.
34. Zheng A, Moritani T. Influence of CoQ10 on autonomic nervous activity and energy metabolism during exercise in healthy subjects. J Nutr Sci Vitaminol (Tokyo). 2008 Aug; 54(4):286-90. PMID: 18797149.
35. Shin KO, Moritani T. The combined effects of capsaicin, green tea extract and chicken essence tablets on human autonomic nervous system activity. J Nutr Sci Vitaminol (Tokyo). 2007 Apr;53(2):145-52. PMID: 17616002.
36. Zheng A, Moritani T. Effect of the combination of ginseng, oriental bezoar and glycyrrhiza on autonomic nervous activity as evaluated by power spectral analysis of HRV and cardiac depolarization-repolarization process. J Nutr Sci Vitaminol (Tokyo). 2008 Apr; 54(2):148-53. PMID: 18490845.
37. Christensen JH, Schmidt EB. Autonomic nervous system, heart rate variability and n-3 fatty acids. J Cardiovasc Med (Hagerstown). 2007 Sep;8 Suppl 1:S19-22. PMID: 17876192.
38. Radaelli A, Cazzaniga M, Viola A, Balestri G, Janetti MB, Signorini MG, Castiglioni P, Azzellino A, Mancia G, Ferrari AU. Enhanced baroreceptor control of the cardiovascular system by polyunsaturated Fatty acids in heart failure patients. J Am Coll Cardiol. 2006 Oct 17;48(8): 1600-6. Epub 2006 Sep 26. PMID: 17045894.
39. Pongchaidecha A, Lailerd N, Boonprasert W, Chattipakorn N.Effects of curcuminoid supplement on cardiac autonomic status in high-fat-induced obese rats. Nutrition. 2009 Jul-Aug;25(7-8):870-8. Epub 2009 Apr 23. PMID: 19398300.
40. Katayama Y, Homma T, Hara Y, Hirai K.Tea catechin, (-)-epigallocatechin gallate, facilitates cholinergic ganglion transmission in the myenteric plexus of the guinea-pig small intestine. Neurosci Lett. 2002 Feb 15;319(2):63-6. PMID: 11825671
41. Watkins LL, Connor KM, Davidson JR. Effect of kava extract on vagal cardiac control in generalized anxiety disorder: preliminary findings. J Psychopharmacol. 2001 Dec; 15(4):283-6. PMID: 11769822.
42. MJ. Lafitte, M. Fèvre-Genoulaz, SS. Srikanta, L. Punitha, S. Vidyanand. 500 heart beats for assessing diabetic autonomic neuropathy. INT. J. DIAB. DEV. COUNTRIES (2005), VOL. 25.
43. Rosenfeldt F, Hilton D, Pepe S, Krum H. Systematic review of effect of coenzyme Q10 in physical exercise, hypertension and heart failure. Biofactors. 2003;18(1-4):91-100.
44. Bonilla DL, Fan YY, Chapkin RS, McMurray DN.Transgenic mice enriched in omega-3 fatty acids are more susceptible to pulmonary tuberculosis: impaired resistance to tuberculosis in fat-1 mice. J Infect Dis. 2010 Feb 1;201(3):399-408. PMID: 20053136.
45. Abriel H, Schläpfer J, Keller DI, Gavillet B, Buclin T, Biollaz J, Stoller R, Kappenberger L. Molecular and clinical determinants of drug-induced long QT syndrome: an iatrogenic channelopathy. Swiss Med Wkly. 2004 Nov 27; 134(47-48):685-94. PMID: 15616901

## Claims

1. Therapeutically effective amounts of vitamin MK-7, for use in a method of increasing High Density Lipoprotein (HDL) in a mammal in need thereof.

## Patentansprüche

1. Therapeutisch wirksame Mengen von Vitamin MK-7 für die Verwendung in einem Verfahren zur Erhöhung von High Density Lipoprotein (HDL) bei einem Säuger, der diese benötigt.

## Revendications

1. Quantités thérapeutiquement efficaces de vitamine MK-7, pour une utilisation dans une méthode d'augmentation des Lipoprotéines de Haute Densité (HDL) chez un mammifère en ayant besoin.
